# FASCICULE DE BREVET EUROPEEN

(11) **EP 2 213 299 B1**
(45) Date de publication et mention de la délivrance du brevet: **09.09.2015**
(21) Numéro de dépôt: 09151596.5
(22) Date de dépôt: 29.01.2009
(51) Int. Cl.: A61K 39/21, A61K 39/12

(54) **Composition vaccinale à base de virus exhibant une protéine à motif(s) en doigt de zinc, son procédé de préparation et son utilisation**
Impfstoffzusammensetzung auf der Basis eines Virus mit Zinkfingermotiv(en), ihr Herstellungsverfahren und ihre Anwendung
Virus-based vaccine composition having a protein with zinc finger pattern(s), method of preparing and using same

(43) Date de publication de la demande: 04.08.2010
(73) Titulaire: Vandevelde, Michel, 1301 Bièrges (BE); Margery, Hélène, 1301 Bièrges (BE)
(72) Inventeur: Vandevelde, Michel, 1301 Bièrges (BE); Margery, Hélène, 1301 Bièrges (BE)
(74) Mandataire: Claeys, Pierre

(56) Documents cités:
- US-A1- 2004 132 785
- MORCOCK D R: "Elimination of retroviral infectivity by N-ethylmaleimide with preservation of functional envelope glycoproteins" JOURNAL OF VIROLOGY, THE AMERICAN SOCIETY FOR MICROBIOLOGY, US, vol. 79, no. 3, 1 février 2005 (2005-02-01), pages 1533-1542, XP009119422 ISSN: 0022-538X
- RICE W G ET AL: "AZODICARONAMIDE INHIBITS HIV-1 REPLICATION BY TARGETING THE NUCLEOCAPSID PROTEIN" NATURE MEDICINE, NATURE PUBLISHING GROUP, NEW YORK, NY, US, vol. 3, no. 3, 1 mars 1997 (1997-03-01), pages 341-345, XP001189942 ISSN: 1078-8956
- LU WEI ET AL: "Therapeutic dendritic-cell vaccine for simian AIDS" NATURE MEDICINE, NATURE PUBLISHING GROUP, NEW YORK, NY, US, vol. 9, no. 1, 1 janvier 2003 (2003-01-01), pages 27-32, XP002366697 ISSN: 1078-8956
- ROSSIO J ET AL: "Inactivation of human immunodeficiency virus type 1 infectivity with preservation of conformational and functional integrity of virion surface proteins" JOURNAL OF VIROLOGY, THE AMERICAN SOCIETY FOR MICROBIOLOGY, US, vol. 72, no. 10, 1 octobre 1998 (1998-10-01), pages 7992-8001, XP002120813 ISSN: 0022-538X
- GARCÍA ET AL: 'Antiviral and virucidal activities against arenaviruses of zinc-finger active compounds.' ANTIVIRAL CHEMISTRY & CHEMOTHERAPY vol. 11, no. 3, 01 Mai 2000, pages 231 - 237, XP055139481 ISSN: 0956-3202
- Robert R Joiner ET AL: "A new powdered agent for flour maturing", Cereal Chem., vol. 40, 1 January 1963 (1963-01-01), pages 539-553, XP055139496,

## Description

La présente invention concerne une composition pour son utilisation en tant que vaccin, à base d'au moins une souche de virus exhibant au moins une protéine à motif(s) en doigt de zinc PZ, comprenant ces virus dans un état inactivé, ces virus étant entiers et présentant une enveloppe virale intacte après inactivation (Voir Mordock et al., Journal of Virology, Feb 2005, p1533-1542), et son procédé de préparation.

Un grand nombre de virus sont connus à ce jour qui exhibent au moins une protéine à motif(s) dits « en doigt de zinc ». Ces protéines sont appelées de manière abrégée protéines PZ.

Comme virus exhibant de telles protéines PZ, on peut citer notamment des rétrovirus, des arénavirus, des filovirus, des hépavirus, des papillomavirus et des virus de l'herpès. Parmi les protéines PZ connues portées par ces virus on peut citer entre autres les protéines NCp7, Tax, NS5A, VP30, IE63, ORF4, UL69, BMLF1, U42, ORF57, ORF3, BICP27, UL54, M69, Z et E6.

La structure en doigt de zinc de ces protéines PZ est indispensable d'une manière ou d'une autre à la réplication des virus pathogènes qui les contiennent. Ces protéines représentent donc une cible de choix pour des molécules capables d'en éjecter les atomes de zinc, car il se produit alors une modification irréversible de leur structure qui inactive le virus attaqué et abolit complètement son pouvoir infectieux.

De nombreuses molécules ont été testées et ont montré un pouvoir d'éjection des atomes de zinc hors des structures en doigts de zinc des protéines PZ, notamment de la protéine NCp7 du virus HIV. Ces molécules sont listées par exemple dans la demande de brevet WO 2003/060098 et l'azodicarbonamide (ADA) en est une (voir aussi W.G. Rice et coll., Nature Medicine 3, 341-345 (1997)).

L'azodicarbonamide (=azobisformamide) est connu depuis très longtemps (1892), sous la forme d'une substance chimique cristalline (v. The Merck Index 13e éd. 2001, p.918). Cette substance est peu soluble dans l'eau et dans la plupart des solvants organiques, à l'exception du N,N-diméthylformamide et du diméthylsulfoxyde (DMSO). Par ADA, il faut entendre, au sens de la présente invention, chacun des isomères cis et trans de cette substance ainsi que leur mélange racémique.

Cette substance a été utilisée comme adjuvant dans la farine alimentaire et comme agent gonflant dans la fabrication de produits plastiques et caoutchouteux.

L'activité potentielle de l'ADA a été observée depuis quelque temps dans différents domaines thérapeutiques, en particulier comme agent thérapeutique actif contre des infections virales, certaines affections cancéreuses et des troubles résultant d'une production pathologique de cytokines (voir par exemple EP-B-0524961, EP-B-0941098, EP-B-1032401 et US-A-5585367). Cette activité a été révélée vis-à-vis du virus du SIDA également, voir par exemple Vandevelde M. et coll., AIDS Res. Hum. Retroviruses 1996, 12, 567 ou Erik De Clercq, Institute for Medical Research, KUL Leuven 2001).

D'autres recherches ont montré l'activité de l'ADA contre des arénavirus (v. par exemple C.C. Garcia et coll., J. Gen. Virol. 2006 May, 87 (Pt 5) : 1217-1228), des virus de l'herpès (v. par exemple WO 2003/060098), le virus de la stomatite vésiculaire VSV (v. US-A-5585367), de nombreux rétrovirus (voir Rice WG, Conf Retroviruses Opportunistic Infect Jan 1997), des virus responsables de cancer du col utérin (être humain) HPV (voir Beerheide W., Journal of the National Cancer Institute, July 21, 1999), des virus provoquant l'immunodéficience du singe macaque et chimpanzé (SIV) (National Institute of Allergy and Infectious Diseases [NIAID ] HIV/OI Searchable Chemical Database http://chemdb2.niaid.nih.gov/struct_search/all/url_search.asp?aids_no=029805).

De nombreux chercheurs se sont par ailleurs lancés dans la recherche de vaccins pour combattre les infections dues à ces virus porteurs de protéine PZ, en particulier dans le domaine de la vaccination contre l'immunodéficience humaine.

Dans le brevet EP-B-0658119, on propose d'utiliser des peptides provenant de souches sauvages de virus HIV pour constituer des vaccins thérapeutiques et prophylactiques. Toutefois, ces protéines séparées de leur support membranaire viral ne possèdent pas l'activité antigénique recherchée. Selon la demande de brevet WO 2005/076001, on propose d'utiliser un polypeptide dérivé de la protéine GP41, ce qui donne lieu au même inconvénient que pour la proposition précédente. On a d'ailleurs remarqué que les virus inactivés par du formaldéhyde ou l'action de la chaleur n'induisaient, dans des préparations vaccinales, qu'une réponse modeste en terme d'anticorps du fait de la modification des protéines immunogènes de l'enveloppe virale.

Dans la demande de brevet US-A-2003/228329 on décrit le développement d'un vecteur viral (adénovirus) portant les motifs de l'HIV. Malheureusement, les données de l'essai clinique avec ce vaccin expérimental se sont avérées décevantes.

Dans la demande de brevet WO 2006/038124, on prévoit une composition contenant un virus HIV entier, inactivé par un traitement chimique par de l'aldithriol-2 (AT-2), cette composition étant utilisée dans la préparation d'un vaccin. Le but de ce traitement est d'inactiver le virus tout en maintenant la structure sauvage de ses protéines. Il est toutefois connu que l'AT-2 est apte à créer des ponts disulfure entre les chromosomes des cellules lymphocytaires humaines. D'autre part, au cours de l'inactivation du virus HIV, l'AT-2 se décompose en 2-thiopyridone qui se comporte de manière imprédictible avec les groupes SH des autres protéines. Des études spectrophotométriques ont montré que la 2-thiopyridone s'associe de manière non covalente avec les protéines portant des groupes SH, réduites ou non.

Il faut enfin remarquer que l'homme de l'art se retrouve devant une difficulté particulière. Il est apparu d'une analyse effectuée par le laboratoire moléculaire du National Institute for Health (NIH) des Etats-Unis que le génome humain contenait les informations pour la production de plusieurs centaines de protéines à motif(s) en doigts de zinc, très utiles.

Le problème à résoudre est donc quadruple : II faut prévoir une composition dans laquelle le virus est inactivé, et donc a perdu son pouvoir infectieux, ce virus permettant toutefois simultanément la stimulation d'anticorps par le sujet auquel la composition est administrée. De plus, la composition doit s'avérer tout à fait acceptable en matière de toxicité et le traitement d'inactivation du virus doit présenter des critères stricts de sélectivité, c'est-à-dire qu'il doit être sans conséquence pour les protéines PZ utiles du sujet auquel la composition est administrée.

Pour résoudre ce problème, on a prévu, suivant l'invention, une composition vaccinale à base d'au moins une souche de virus exhibant au moins une protéine à motif(s) en doigt de zinc PZ, caractérisée en ce que ledit zinc de ladite protéine PZ a été éjecté de ladite au moins une protéine PZ par une réaction d'oxydoréduction avec de l'azodicarbonamide et en ce que ladite composition comprend en outre de la biurée formée à partir de l'azodicarbonamide au cours de ladite réaction d'oxydoréduction, ainsi qu'éventuellement un reliquat d'azodicarbonamide n'ayant pas réagi.

Par virus entier, il faut entendre au sens de la présente invention, que le virus inactivé comporte encore la totalité de son enveloppe ou membrane virale, sa nucléocapside et son matériel génétique. Par enveloppe ou membrane virale intacte, il faut entendre que les protéines qui la constituent n'ont été ni chimiquement ni physiquement modifiées par l'inactivation et donc que les motifs antigéniques qu'elles portent sont inaltérés, l'enveloppe virale présentant une conformation et une activité fusiogénique conservées.

Une mesure de l'état d'inactivation du virus peut être effectuée selon l'enseignement notamment de HIV: A Practical Approach Par Jonathan Karn Publié par Oxford University Press, 1995 pp120-125.

Dans cette application l'important est de préciser le pouvoir contaminant des virus après traitement.

Le titre initial du virus est exprimé comme le nombre de doses infectieuses (nombre prédéterminé de particules infectieuses pour chaque espèce) pour une culture par millilitre de plasma.

Pour cela on utilise habituellement des dilutions sérielles et on vérifie après chaque dilution le pouvoir contaminant des virus sur des cultures de cellules humaines jusqu'à ce que la dilution ne permette plus d'obtenir une dose de virus suffisante pour l'infection.

Les résultats sont exprimés en TCID50 (dose infectant 50 p. 100 des puits). Une TCID50, dans le cas du virus HIV, est à peu près équivalente à une particule virale par ml de plasma.

Compte tenu des réductions de titres obtenues dans les différentes expérimentations il a été possible de déterminer la dose de réduction décimale (D10). Une quantité de 3 µg/ml d'ADA diminue de 100. 000 fois le titre infectieux (réduction : 5 logs (plus d'infectivité décelée) après 2 heures d'exposition à 37°C.

Avantageusement, l'inactivation se fait à l'aide d'ADA micronisé. Par ADA micronisé, il faut entendre que cette substance se présente sous la forme de particules d'une taille suffisamment petite pour pouvoir traverser l'enveloppe virale sans nécessiter une dissolution dans un véhicule et cela de manière à maintenir l'enveloppe virale intacte. L'ADA micronisé peut toutefois pénétrer dans le virus en étant en suspension et partiellement en solution dans la phase aqueuse du milieu de culture de ce dernier, ce qui permet de ne pas devoir introduire de solvants pharmaceutiquement peu recommandables, sinon inacceptables dans la composition. En effet, le DMSO jusqu'à présent utilisé dans les expérimentations sur virus avec de l'ADA est reconnu comme pouvant interagir avec les protéines de membrane du virus (Tjernberg et al. J Biomol Screen, 2006), il est aussi connu pour être un agent qui déstructure efficacement les membranes cellulaires pour favoriser la pénétration d'un milieu donné à l'intérieur de celles-ci. C'est entre autres pour cette raison qu'il est largement utilisé comme véhicule pour de nombreux médicaments devant passer par voie transmembranaire. Une utilisation de DMSO à une concentration égale ou inférieure à 0,5 %, de préférence inférieure à 0,3 % en volume est acceptable.

Suivant une forme de l'invention, l'azodicarbonamide micronisé se présente sous la forme de particules qui sont dépourvues d'additif, c'est-à-dire en particulier sans adjuvant tensio-actif ou enrobage, et qui montrent une taille inférieure à 10 µm, de préférence à 8 µm, en particulier à 5 µm. Avantageusement, lesdites particules présentent une taille moyenne d₅₀ inférieure à 2 µm et un d₉₀ inférieur à 4 µm.

Cette granulométrie étroite permet une reproductibilité très poussée des résultats obtenus. On peut par exemple préparer un ADA de ce genre selon l'enseignement de la demande de brevet WO 2007/048820.

Lors de la réaction d'oxydo-réduction précitée, l'ADA en présence de protons issus de protéines PZ se décompose en biurée de la manière suivante :

NH₂-CO-N=N-CO-NH₂ + 2 H⁺ → NH₂-CO-NH-NH-CO-NH₂

Par exemple, dans le cas du virus HIV et de sa protéine de nucléocapside NCp7 comprenant un doigt de zinc, l'ADA pénètre dans les particules virales, au travers de l'enveloppe du rétrovirus et interagit avec la protéine NCp7 (voir Rice WG et al; Nat Med. March 1997).

La protéine NCp7 contient deux copies d'un fragment peptidique présentant la séquence suivante Cys-X2-Cys-X4-His-X4-Cys (aussi appelé en abrégé CCHC), coordonnée avec un ion métallique de zinc (II). De ceci résulte une conformation tridimensionnelle de la protéine NCp7 avec deux doigts de zinc. Chaque doigt de zinc présente trois liaisons Zn-S et une liaison Zn-N qui contribuent toutes quatre à la conformation tridimensionnelle en doigt de zinc.

L'azodicarbonamide étant un donneur d'électrons grâce à sa liaison N=N, il permet de déstabiliser la liaison Zn-S. Ceci a pour résultat la formation d'un pont disulfure entre les deux atomes de soufre adjacents de la structure tridimensionnelle et l'élimination d'un groupe moléculaire thiolate correspondant hors du site de liaison à l'ion métallique ainsi qu'une éjection de zinc.

Après avoir donné ses électrons de la liaison N=N, l'azodicarbonamide est réduit en biurée par l'acceptation de deux protons transformant le groupement -N=N- en un groupement -NH-NH-.

Dans ce cadre, les réactions d'ADA avec NCp7 mènent à une déstabilisation de la sphère de coordination du zinc dans la NCp7 qui est suivie par l'inhibition de l'HIV (I.A. Topol et coll., J. Phys. Chem. B 2001, 105, 11341-11350).

La protéine NCp7 est critique pour la réplication virale et, de ce fait, la modification de sa conformation tridimensionnelle ne permet plus à la protéine NCp7 de reconnaître l'enzyme de la transcription inverse (RT) et la réplication du virus n'est plus possible.

Etant donné que l'azodicarbonamide n'a un effet que sur la protéine NCp7 se trouvant dans la nucléocapside des particules virales du virus HIV, la conformation initiale du virus (extérieure et générale) est conservée ainsi que l'activité fusiogénique de la protéine d'enveloppe GP120 alors que le zinc est éjecté de la protéine PZ déprotonisée.

Un phénomène similaire a été observé sur d'autres protéines telles que les protéines E6, NS5A, IE63 lors de l'action de l'ADA, entre autres sur les virus HPV, HCV et HSV.

La biurée est le seul catabolite de l'ADA in vivo (Concise International Chemical Assessment Document 16 ; World Health Organization : Geneva, 1999). De plus, dans des conditions expérimentales correspondant au corps humain (pH neutre, température de 37° C, milieu sérique humain) il est apparu que la réaction indiquée ci-dessus était irréversible. D'autre part, la rapide transformation de l'ADA en biurée en présence de cellules ou de sang est à présent vérifiée et donc on peut en déduire que le reliquat d'ADA n'ayant pas réagi pendant l'oxydo-réduction va se décomposer en biurée en très peu de temps et de manière irréversible dans le corps du sujet auquel on a administré la composition. Dès que cette rapide décomposition du reliquat d'ADA en biurée a eu lieu, il ne subsiste plus de la composition administrée aucun agent susceptible d'interagir avec des protéines PZ utiles qui sont présentes dans le corps du sujet traité. Enfin, il est apparu, après de multiples essais sur animaux et sur volontaires sains, que l'absorption d'ADA et de biurée ne pouvait mettre en évidence des problèmes de toxicité ou de tolérance qu'à des concentrations anormalement et inutilement élevées. A ce jour l'azodicarbonamide dispose donc de données toxicologiques, en particulier concernant son unique catabolite, la biurée, qui sont suffisantes pour l'administration à des volontaires sains ou malades.

La composition selon l'invention comprend donc, outre des virus inactivés entiers dans lesquels le zinc a été éjecté de ladite au moins une protéine PZ et l'enveloppe virale est intacte, de la biurée ainsi qu'éventuellement un reliquat d'azodicarbonamide en excès et n'ayant donc pas réagi. La biurée se trouve au sein même de l'enveloppe virale, puisque dans la composition selon l'invention, l'ADA pénètre aisément au sein de celle-ci pour y être réduit. Toutefois, de l'ADA en excès peut encore se trouver à l'extérieur et/ou à l'intérieur des enveloppes virales

Suivant une forme de réalisation avantageuse de l'invention, la composition contient en outre un donneur de protons pharmaceutiquement compatible, dans un état déprotonisé qui est également pharmaceutiquement compatible et qui résulte d'une réaction d'oxydoréduction additionnelle avec ledit reliquat d'azodicarbonamide n'ayant pas réagi, ainsi qu'éventuellement
- un reste de donneur de protons n'ayant pas réagi.

Dans le but d'éviter la présence même temporaire d'ADA dans le corps du sujet traité, et donc d'empêcher tout risque d'attaque de protéine PZ utile par un reliquat d'ADA dans la composition suivant l'invention, on introduit dans celle-ci, avant administration, ledit donneur de protons en une quantité suffisante pour décomposer le reliquat d'ADA en biurée. La composition vaccinale selon l'invention est alors dépourvue d'ADA. Avantageusement, le donneur de protons est un réducteur physiologique, de préférence une protéine exhibant au moins un groupe sulfhydryle libre, par exemple choisi parmi le groupe constitué de la cystéine, du glutathion, du nicotinamide phosphate et de leurs dérivés et mélanges et en ce que le donneur de protons déprotonisé est respectivement la cystine, le glutathion oxydé ou le NADP⁺ (forme oxydée du nicotinamide phosphat) ou un dérivé de ceux-ci.

Préférentiellement, le donneur de protons est la cystéine ou un dérivé de celle-ci, par exemple de la N-acétyl-L-cystéine, et le donneur de protons déprotonisé est la cystine ou un dérivé de celle-ci. La cystéine et la cystine sont des protéines tout à fait courantes dans le corps humain ou animal et elles ne présentent donc aucun danger en matière de toxicité et de tolérance.

Suivant encore une autre forme de réalisation de l'invention, la composition contient en outre au moins un adjuvant pharmaceutiquement acceptable, tel que l'adjuvant de Freund complet ou incomplet, l'alun ou encore d'autres composés tels que décrits dans Vermount et coll. Ann. Méd. Vét., 2003 147; p 393-401.

De cette façon, la composition vaccinale selon l'invention est susceptible de déclencher une réponse immunitaire améliorée par la présence des adjuvants, une fois administrée à un patient.

Dans une forme de réalisation avantageuse, la composition vaccinale selon l'invention comprend en outre des cellules immunocompétentes isolées qui ont éventuellement au moins partiellement internalisé lesdits virus inactivés.

Par cellules immunocompétentes, on peut entendre, suivant l'invention, par exemple des cellules dendritiques, matures ou immatures, des cellules de Langerhans, des phagocytes (monocytes et macrophages), ou encore des cellules endothéliales ou épithéliales exprimant des molécules du complexe majeur d'histocompatibilité CMH de classe 2. Ces cellules peuvent être en différents états de différenciation connus en soi qui ne seront pas détaillés ici.

Au sens de l'invention, lorsque la composition vaccinale selon l'invention comprend en outre des cellules immunocompétentes isolées, il est possible que les virus inactivés se trouvent dans lesdites cellules immunocompétentes isolées, si l'internalisation du virus a déjà eu lieu, ou ne se trouvent pas encore dans ces cellules si cette internalisation ne s'est pas encore produite.

La présente invention concerne en outre un procédé de préparation d'une composition suivant l'invention, comprenant:
- une mise en contact d'au moins une souche de virus exhibant au moins une protéine à motif(s) en doigt de zinc PZ avec de l'azodicarbonamide micronisé,
- une pénétration de l'azodicarbonamide micronisé à travers l'enveloppe virale desdits virus en laissant celle-ci intacte, et
- une inactivation des virus par une réaction d'oxydoréduction entre l'azodicarbonamide micronisé et ladite au moins une protéine PZ, résultant en une éjection du zinc à partir de ladite protéine PZ et en une décomposition au moins partielle de l'azodicarbonamide en biurée.

Suivant un mode préféré de réalisation de l'invention, ce procédé comprend en outre
- une mise en contact des virus inactivés avec un donneur de protons pharmaceutiquement acceptable, qui est aussi pharmaceutiquement acceptable à l'état déprotonisé, et
- une réaction d'oxydoréduction additionnelle entre ce donneur de protons et un éventuel reliquat d'azodicarbonamide n'ayant pas réagi, ce qui déprotonise ledit donneur de protons et décompose ledit reliquat en biurée.

Suivant un mode avantageux de réalisation du procédé, celui-ci comprend en outre une élimination du zinc éjecté, par tout moyen approprié connu de l'homme de métier, par exemple par centrifugation. On peut ensuite prévoir un isolement des virus inactivés, par tout moyen approprié connu de l'homme de métier, par exemple par ultracentrifugation, de manière à éliminer au maximum de la composition tout composant autre que ces virus inactivés.

Dans une forme avantageuse du procédé selon l'invention, le procédé comprend en outre une impulsion de cellules immunocompétentes isolées par ladite composition contenant des virus inactivés par azodicarbonamide, une mise en culture ex vivo des cellules impulsées et une expression à leur surface d'antigènes spécifiques desdits virus inactivés.

L'invention concerne aussi l'utilisation d'une composition vaccinale suivant l'invention comme vaccin prophylactique ou thérapeutique contre au moins une infection par au moins un desdits virus exhibant au moins une protéine à motif(s) en doigt de zinc. Le vaccin produit est avantageusement administrable par injection ou par voie transmuqueuse, orale, génitale ou rectale.

La composition suivant l'invention ou produite selon un procédé suivant l'invention peut être utilisée telle quelle comme vaccin. Le virus inactivé peut être autologue ou non. Le vaccin peut aussi être formé d'un cocktail de différents virus inactivés.

Un vaccin thérapeutique à base de virus inactivé, de préférence autologue, et présentant selon l'invention une conformation intacte permet d'amplifier, par le virus inactivé, la réponse immunitaire déficiente d'un patient vis-à-vis de l'infection et donc de lutter efficacement contre celle-ci puisque les composants du système immunitaire qui pourront lutter efficacement contre le virus inactivé pourront également lutter contre le virus actif.

Le vaccin prophylactique est quant à lui plutôt préventif et son but principal est d'induire la production par l'organisme d'anticorps et de cellules cytotoxiques dirigées vers les éléments présentant le motif antigénique. Il est donc essentiel que les motifs antigéniques du virus inactivé du vaccin selon l'invention soient conservés après le traitement d'inactivation afin de s'assurer que les défenses immunitaires orientées vers ses motifs antigéniques spécifiques puissent aussi reconnaître les motifs antigéniques du virus infectieux et ainsi neutraliser l'infection par ce même virus par la cascade du complément du système immunitaire et/ou par la destruction des cellules infectées présentant à leur surface lesdits motifs antigéniques grâce aux lymphocytes cytotoxiques (CTL).

Avantageusement, le vaccin produit est un vaccin administrable par injection ce qui permet d'intensifier les défenses de la personne déjà porteuse du virus ou par voie transmuqueuse, orale, génitale ou rectale.

L'induction d'une forte réponse immune à la porte d'entrée à l'encontre de l'agent infectieux semble être la voie la plus appropriée pour prévenir une infection par des agents viraux Human papilloma virus, hépatites, VIH, herpes etc. Par conséquent, la stratégie permettant d'induire une réponse immune protectrice au niveau de la muqueuse est un aspect critique du développement de nouveaux vaccins.

Des ovules vaginaux préparés suivant les règles pharmaceutiques (pH, temps de dissolution, couleur, parfum) ainsi que des suppositoires spécifiques à la protection de la muqueuse ont plus de chance d'être protecteurs. En combinaison avec une administration par injection sous-cutanée la voie muqueuse est la plus proche du phénomène physiologique infectieux.

L'utilisation suivant l'invention peut donc comprendre une impulsion de cellules immunocompétentes isolées, prélevées d'un sujet porteur de virus, ou d'un sujet sain, par ladite composition contenant des virus inactivés par azodicarbonamide, une mise en culture ex vivo des cellules impulsées, en présence notamment de cytokines d'activation, et une expression à leur surface d'antigènes spécifiques desdits virus inactivés.

Les cellules immunocompétentes ainsi cultivées ex vivo en présence de virus inactivé selon l'invention internalisent le virus inactivé et sont alors dites chargées ou stimulées et prêtes à être ré-injectées par voie d'administration sous-cutanée, intradermique ou intralymphatique. Ces cellules immunocompétentes sont de préférence activées par l'action de cytokines et expriment à leur surface des antigènes (peptides) spécifiques du virus inactivé internalisé ayant permis de les stimuler.

Les cellules immunocompétentes agissent sur les lymphocytes T cytotoxiques (Tc) et T helper (Th) en les activant et en ayant un rôle sur leur prolifération grâce à une cascade de réactions de la réponse immunitaire déclenchée par une multitude de molécules exprimées par les cellules immunocompétentes. Ces cellules sont alors d'excellentes cellules activatrices des lymphocytes T cytotoxiques spécifiques du virus inactivé selon l'invention par exemple pour un patient sain, mais aussi pour un patient infecté.

Dans le cas de sujets infectés, il existe un effet d'amplification de la réaction immune face au virus inactivé selon l'invention, permettant également de lutter contre l'infection présente. En effet, chez le porteur de virus ou patient infecté, les cellules dendritiques sont généralement en nombre réduit et la plupart du temps, ces cellules immunocompétentes sont déjà porteuse de virus infectieux. L'utilisation de cellules dendritiques préalablement traitées par la composition vaccinale selon l'invention permet d'introduire des cellules immunocompétentes qui ne sont pas infectées et qui permettent d'amplifier par le virus inactivé la réponse immunitaire vis-à-vis dudit virus actif.

C'est pourquoi dans le cas du virus HIV, les cellules dendritiques sont obtenues à partir de monocytes ne présentant pas de récepteurs CD4 (porte d'entrée pour le virus). Ces cellules sont donc saines et par une cascade de stimulation, les monocytes sont ensuite différencié en cellules dendritiques saines qui seront alors chargées avec du virus inactivé.

L'invention concerne également une méthode de traitement d'un individu infecté par un virus exhibant au moins une protéine à motif(s) en doigt de zinc PZ, cette méthode comprenant une administration, sous forme d'un vaccin thérapeutique, d'une composition suivant l'invention ou de cellules immunocompétentes traitées par une composition suivant l'invention.

On peut aussi prévoir suivant l'invention une méthode de traitement d'un individu sain comprenant une administration, sous forme d'un vaccin prophylactique, d'une composition suivant l'invention ou de cellules immunocompétentes isolées traitées par une composition vaccinale suivant l'invention.

D'autres particularités concernant la composition, le procédé et l'utilisation de l'invention sont indiquées dans les revendications.

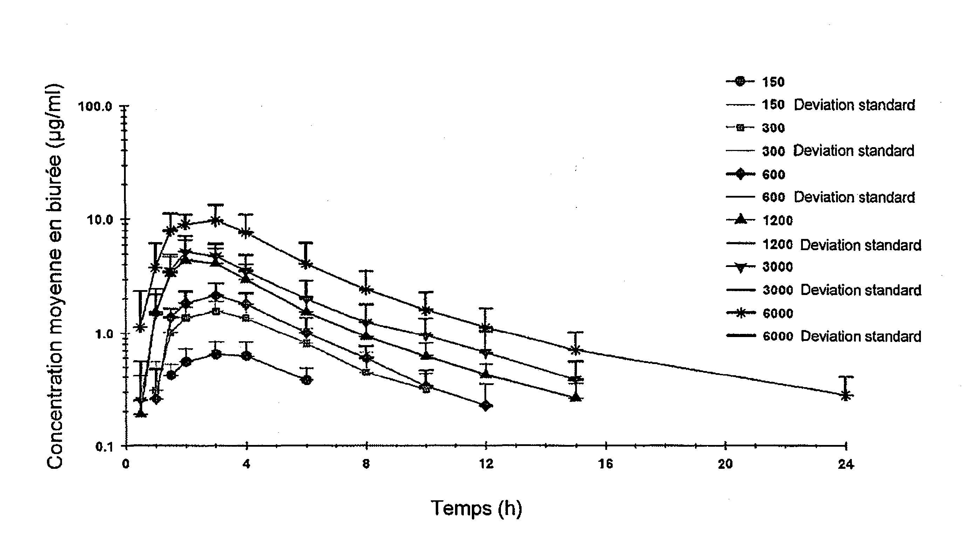

L'invention va à présent être décrite de manière plus détaillée à l'aide des exemples donnés ci-après et en faisant référence à la figure unique annexée qui illustre l'évolution de la concentration plasmique moyenne de la biurée au cours du temps.

### EXEMPLES.-

Dans les exemples, l'azodicarbonamide utilisé a été produit selon le protocole décrit dans la demande de brevet WO2007/048820 au nom de la demanderesse.

### EXEMPLE 1.- Analyse de toxicité de l'ADA et de la biurée (BIU) obtenue lors de la dégradation de l'ADA.

Avant de pouvoir déterminer la toxicité des composés, il s'agit de valider une méthode de dosage de la biurée.

### 1.1 Méthode de dosage

La méthode suivante de détermination du taux de biurée dans des échantillons de plasma hépariné ainsi que dans les urines pour des concentrations qui varient de 0,2 à 20 µg / ml a été considérée comme étant conforme aux recommandations de l'OCDE (Organisation pour la Coopération Économique et le Développement), aux bonnes pratiques de laboratoires (GLP) et avec l'ouvrage "Guidance for Industry: Bioanalytical method validation".

Les produits de séparation (analytes) des échantillons de plasma ou d'urine sont détectés par spectrométrie de masse (MS-MS). La limite inférieure de détection (LOQ) de la biurée est de 0,2 µg / ml alors que la limite supérieure de dégradation LOD est de 20 µg / ml. Les courbes de calibrage sont linéaires de 0,2 à 20 µg / ml avec un coefficient de détermination (r²) de 0,99.

Aucune variation des concentrations de biurée dans les échantillons de plasma ou d'urine n'apparaissait après 55 heures de conservation à température ambiante.

Les concentrations initiales en biurée sont retrouvées dans des échantillons de plasma ou d'urine stockés à -20°C durant 3 mois, et ceux-ci peuvent subir au moins trois cycles de gel / dégel.

### 1.2 Transformation de l'ADA en BIU

La rapide transformation de l'ADA en BIU en présence de cellules ou de sang est connue depuis longtemps et a été confirmée dans des essais sur différents composants du sang exposés à de l'ADA radio-marqué. Seule la BIU a été retrouvée, comme dérivé de cet ADA radio-marqué.

En outre, des doses croissantes d'azodicarbonamide suspendues dans un hydrogel de carboxyméthylcellulose (CMC) à 0,5 % ont été administrées par voie orale à des groupes de 3 souris (poids moyen: 20 g). Un groupe témoin a reçu le véhicule CMC et des échantillons de sang ont été pris 30, 60 et 120 min après administration.

**Tableau 1**

| groupe | [ADA] administrée | [ADA] après 30 minutes | [ADA] après 60 minutes | [ADA] après 120 minutes | [BIU] après 60 minutes |
|---|---|---|---|---|---|
| 1 | 1,25 mg, soit 62,5 mg/kg | néant | néant | néant | 3 µg/ml |
| 2 | 2,50 mg, soit 125 mg/kg | néant | néant | néant | 5µg/ml |
| 3 | 5,00 mg, soit 250 mg/kg | néant | néant | néant | 8 µg/ml |
| 4 | 10 mg soit 500 mg/kg | néant | néant | néant | 10 µg/ml |
| 5 | 0 mg | néant | néant | néant | néant |

Ensuite, le même test a été réalisé sur des rats afin de déterminer si la conversion de l'ADA en biurée était différente en fonction du sexe de l'animal et aucune différence due au sexe n'a été observée.

### 1.3 Précautions préalables: tests précliniques

De nombreux tests ont été menés dans la phase préclinique de l'azodicarbonamide en se référant à chaque fois à un groupe témoin ne recevant que le véhicule et les conclusions suivantes ont pu être tirées:
1. Aucune différence significative entre animaux traités et contrôles n'a été observée en matière de poids corporel, la quantité de nourriture absorbée par les animaux étant identique.
2. La toxicité oculaire a été suivie pour écarter un éventuel dépôt de cristaux de BIU et aucune différence n'a été observée entre groupes traités et groupe témoin.
3. Puisque les protéines à motif en doigts de zinc peuvent jouer un rôle dans l'apparition du diabète en particulier la protéine « ZAC », les effets sur le taux de glucose sanguin et sur le taux d'insuline ont été étudiés durant cette période. Le test de tolérance au glucose ne différait pas entre le groupe ADA et le groupe témoin tant pour ce qui concerne les dosages de glucose que d'insuline.
4. Etant donné que l'appareil reproducteur des mammifères est extrêmement sensible à l'intégralité des protéines PZ, une étude préclinique a également analysé l'indice de fertilité (nombre de rapports nécessaires pour obtenir une fécondation), indice de gestation (nombre d'individus par portée), indice de survie (nombre d'individus nouveau-nés survivant 4 jours ou plus), indice de lactation (nombre d'individus nouveau-nés survivant 4 jours ou plus dans la portée conservée) et ce pour trois générations. Aucun effet en matière de reproduction n'a été observé.
5. L'effet de l'ADA entraînant une concentration sanguine en BIU supérieure à 20µg/ml a été également évalué sur la tension artérielle, le rythme cardiaque, et l'électrocardiogramme. Des concentrations sanguines en BIU supérieures à 20 µg/ml n'induisent aucune différence significative sur les paramètres étudiés.
6. L'effet de l'ADA entraînant une concentration sanguine en BIU supérieure à 20µg/ml a aussi été évalué sur la tension artérielle, et le système respiratoire (fréquence, pic de volume inspiratoire, pic de volume expiratoire, temps d'inspiration et d'expiration, variation de volume après administration de doses croissantes d'ADA). Ces doses n'induisent aucune différence significative sur les paramètres étudiés.
7. L'effet de l'ADA entraînant une concentration sanguine en BIU supérieure à 20µg/ml (limite de détection de la méthode) a été évalué sur le système nerveux central par la batterie d'observations standardisées « Irwin » ainsi que la température corporelle. Ces concentrations sanguines en BIU n'induisent aucune différence significative sur les paramètres étudiés.
8. On a mesuré les interactions potentielles de la BIU avec le cytochrome humain P450 3A et la BIU n'a inhibé aucune des enzymes du cytochrome P450 3A (CYP3A) jusqu'à une dose de 100µM.
9. La biurée a également été testée dans le cadre d'un essai de mutation inverse de souches Salmonella typhimurium H-Histidine et Escherichia coli WP2 (uvrA) (pKM 101). Les résultats ont été négatifs.

### 1.4 Etude de l'effet de doses simples et croissantes chez des volontaires humains sains

La méthode de dosage de la biurée (BIU) sanguine a été validée pour des concentrations supérieures à 20µg/ml par dilution sérielle.

La pharmacocinétique de la biurée (BIU), comme marqueur après ingestion de doses croissantes d'azodicarbonamide (ADA), a été étudiée chez 18 volontaires sains (sexe masculin : âgés de 18 à 45 ans) répartis en plusieurs groupes dont deux groupes placebos.

Lors de la première période, les volontaires ont été divisés en groupes de 6 personnes (4 actifs, 2 placebos par groupe) recevant : groupe A une dose de 150 mg (2,15 mg/kg) ; groupe B une dose de 600 mg (8,5mg/kg); groupe C une dose de 3000 mg (42,5mg/kg).

Lors de la seconde période (7 jours sans traitement (« wash-out ») lesdits volontaires ont été divisés en groupes de 6 personnes (4 actifs, 2 placebos par groupe) recevant : groupe A1 une dose de 300 mg (4,3 mg/kg) ; groupe B1 une dose de 1200 mg (17mg/kg) ; groupe C1une dose de 6000 mg (85 mg/kg).

Comme on peut le voir à la figure, les concentrations plasmatiques maximales individuelles de BIU ont été atteintes dans les 4 heures qui ont suivi la prise de la dose, avec des valeurs du « tₘₐₓ» moyen entre 2,1 et 3,2 h pour les niveaux de dose différents; le temps du décalage et l'écart entre le temps d'ingestion et le temps de concentration maximale diminue avec l'importance de la quantité d'ADA absorbée.

Les valeurs moyennes de concentration sanguine maximale de BIU sont illustrées au tableau 2.

**Tableau 2**

| Groupe | concentration sanguine maximale de BIU |
|---|---|
| A (150 mg ADA) | 0,648 µg/ml |
| A1 (300mg ADA) | 1,556 µg/ml |
| B (600mg ADA) | 2,144 µg/ml |
| B1 (1200 mg ADA) | 4,89 µg/ml |
| C (3000 mg ADA) | 5,182 µg/ml |
| C1 (6000 mg ADA) | 8,995 µg/ml |

La biurée en concentration sérique dans des limites de 0,648 µg/ml à 8,995 µg/ml a été très bien tolérée et a été prouvée comme étant sûre après évaluation des données cliniques et de laboratoire incluant les valeurs de méthémoglobine, des signes vitaux en ce compris les électrocardiogrammes (espace QT) et toutes les données analytiques concernant la fonction rénale.

L'incidence d'effets secondaires est la même que dans le groupe placebo.

### EXEMPLE 2.- Irréversibilité de la réaction de réduction de l'azodicarbonamide en biurée.

Le comportement redox de l'ADA et de la BIU au niveau d'électrodes solides par voltamétrie cyclique a été évalué.

La BIU s'oxyde en milieu acide à des potentiels positifs assez élevés (supérieurs à 1 volt par rapport à Ag/AgCl KCI 3 M) avec production d'ADA, tandis que l'ADA est réductible à pH neutre à des potentiels proches de 0,2 V Ag/AgCl KCI 3 M.

La biurée a été mise en contact avec des systèmes oxydants expérimentaux de peroxydase de raifort (HRP)/H₂O₂, de myéloperoxydase/CI⁻ /H₂O₂, et de cytochrome P450/NADH afin d'étudier la ré-oxydation éventuelle de la biurée en azodicarbonamide. Les mesures ont été effectuées avec les enzymes libres en solution mais également avec les enzymes immobilisées sur électrode solide.

Dans les conditions expérimentales suivantes : pH neutre, 37°C et milieu sérique humain, la transformation d'ADA en BIU est irréversible, même en présence des enzymes oxydantes les plus puissantes.

### EXEMPLE 3.- Dégradation de l'ADA en BIU par l'addition d'un donneur de protons.

L'électro-activité de l'ADA (0,2 V) au niveau des électrodes solides a été exploitée afin de suivre, en temps réel, par un chrono-ampèremètre, sa disparition rapide en contact avec un fluide biologique.

La mesure électrochimique n'étant pas perturbée par la turbidité du milieu (plasma, culture de cellule), il a été possible d'opérer directement sur les échantillons biologiques sans traitement de ceux-ci (autre qu'une simple dilution). Une électrode de carbone vitreux polarisée à -0,2 V positionnée dans le milieu de mesure a permis le suivi chrono-ampérométrique de l'ADA.

Les substances consommant l'ADA provoquent une chute du courant de réduction. Cette chute de courant est proportionnelle au composé ajouté. L'effet d'une série de réducteurs physiologiques a été étudié : un dérivé de cystéine, la N-acétyl cystéine, le nicotinamide phosphate et le glutathion.

### 3.1 Effet des substances réductrices sur la concentration d'ADA

Comme on vient de le mentionner plus haut, les substances consommant l'ADA provoquent une chute du courant de réduction proportionnelle au composé ajouté. Dans le tableau 3, les effets de la cystéine, plus particulièrement un dérivé de cystéine, la N-acétyl cystéine et du glutathion ont été testés.

On a utilisé un tampon phosphate PBS à pH 7,4 pour réaliser les mesures ampérométriques et une concentration d'ADA de 2.10⁻⁵ mole. On a ensuite ajouté des concentrations croissantes de cystéine et de glutathion pour obtenir respectivement une concentration finale de 1.10⁻⁵ mole, de 2.10⁻⁵ mole, et de 3.10⁻⁵ mole de cystéine et de glutathion.

**Tableau 3**

| Composition | Courant de réduction (après 1 min) | Composition | Courant de réduction (après 1 min) |
|---|---|---|---|
| PBS seul | 0,15 mA | PBS seul | 0,05 mA |
| (contrôle négatif) | | (contrôle négatif) | |
| PBS + ADA | 0,6 mA | PBS + ADA | 0,42 mA |
| (contrôle positif) | | (contrôle positif) | |
| PBS + ADA + | 0,4 mA | PBS + ADA + | 0,27 mA |
| 1.10⁻⁵ mole Cys | | 1.10⁻⁵ mole GSH | |
| PBS + ADA + | 0,25 mA | PBS + ADA + | 0,17 mA |
| 2.10⁻⁵ mole Cys | | 2.10⁻⁵ mole GSH | |
| PBS + ADA + | 0,17 mA | PBS + ADA + | 0,10 mA |
| 3.10⁻⁵ mole Cys | | 3.10⁻⁵ mole GSH | |

De manière surprenante, c'est l'ajout de cystéine qui entraîne la réduction la plus rapide et la plus forte de ADA en BIU (H-Phar- ULB - Institut de pharmacie Prof Kauffman 2007).

### 3.2 Effet de la cystéine sur l'ADA en condition physiologique "in vitro"

On a utilisé un tampon phosphate PBS à pH 7,4 pour réaliser les mesures ampérométriques et une concentration d'ADA de 10⁻³ mole. Afin de reproduire au mieux le milieu sanguin dans lequel l'ADA est rapidement dégradé de manière naturelle, 50 µl de globules rouges (environ 5000 cellules) ont été ajoutés. On a ensuite ajouté 10⁻⁴ moles de cystéine. Les mesures du courant de réduction sont illustrées au tableau 4 ci-dessous.

**Tableau 4**

| N° | Composition | Courant de réduction |
|---|---|---|
| 1 | PBS seul (contrôle négatif) | 0,05 mA |
| 2 | PBS + ADA + Globules rouges (contrôle positif- t = o) | 0,6 mA |
| 3 | PBS + ADA + Globules rouges (t = 5 min) | 0,32 mA |
| 4 | PBS + ADA + Globules rouges (t = 5 min) + cystéine | 0,05 mA |

Comme on peut le constater, entre les échantillons n°2 et 3, le courant de réduction diminue de 0,6 à 0,32 mA illustrant la dégradation naturelle de l'ADA dans le système sanguin. La concentration de cystéine ajoutée dans l'échantillon 4 a permis de retrouver la valeur de courant de réduction de 0,05 mA (valeur du tampon phosphate seul PBS) montrant clairement que tout l'ADA a été dégradé.

### 3.3 Effet de la concentration de la cystéine sur la dégradation sanguine de l'ADA

On a utilisé un tampon phosphate PBS à pH 7,4 pour réaliser les mesures ampérométriques et une concentration d'ADA de 2.10⁻⁵ mole. On a ensuite ajouté des concentrations croissantes de cystéine pour obtenir respectivement une concentration finale de 1.10⁻⁵ mole, de 2.10⁻⁵ mole, et de 3.10⁻⁵ mole de cystéine. Les mesures du courant de réduction sont illustrées au tableau 5 ci-dessous.

**Tableau 5**

| N° | Composition | Courant de réduction après 1 min |
|---|---|---|
| 1 | PBS seul (contrôle négatif) | 0,15 mA |
| 2 | PBS + ADA (contrôle positif) | 0,6 mA |
| 3 | PBS + ADA + 1.10⁻⁵ mole Cys | 0,4 mA |
| 4 | PBS + ADA + 2.10⁻⁵ mole Cys | 0,25 mA |
| 5 | PBS + ADA + 3.10⁻⁵ mole Cys | 0,17 mA |

Comme on peut le voir du tableau 4, au rapport de 3 molécules de cystéine pour 2 molécules d'ADA, le passage de courant dans la solution est pratiquement ramené à son point initial dans la minute qui suit l'adition de cystéine, ce qui confirme le modèle théorique suggérant qu'il faut deux molécules de cystéine pour obtenir une réduction complète d'une molécule d'ADA.

### Exemple 4.- Production de virus HIV inactivé par de l'ADA micronisé

### 4.1 Production de virus HIV en culture à petite échelle:

Le virus est obtenu par la mise en culture de particules virales recueillies lors d'une prise de sang selon un protocole standard tel que celui décrit par Goebel et coll. (AIDS 2001 vol.15 - pp 33-45) sous la rubrique « Ex vivo HIV-1 cultures ».

On a extrait les virus du patient à traiter grâce à une co-culture de ses propres lymphocytes T CD4+ infectés en présence de monocytes périphériques (cellules PBMC) obtenus à partir du sang du patient prélevé. Ensuite, les lymphocytes T CD4+ infectés ont été activés par de la phytohémagglutinine (Sigma, St. Louis, Mo.USA).

Tous les 7 jours, un échantillon de surnageant a été prélevé pour mesurer le taux de p24 et ainsi évaluer la croissance virale. Lorsque le taux de p24 était de 50 µg/ml, la culture de cellules a été centrifugée et le surnageant stocké en lots de stock de virus à -80°C de manière à obtenir, après ultracentrifugation, 2.10¹⁰ particules virales par ml.

### 4.2 Production de virus HIV inactivé

La solution stock de particules virales congelées à -80°C est traitée pendant 2 heures à 37°C par une concentration de 0,5 mM d'azodicarbonamide (ADA) micronisé (HPH116 de la firme H-Phar Belgique) qui, comme décrit ci-avant, détruit la protéine à doigt de zinc n°7 de la nucléocapside virale. La composition virale est traitée de manière telle que la quantité de virus désactivé par l'ADA entraîne la présence de 200 ng/ml de p24 provenant du virus HIV soit spécifique au patient lorsque le virus obtenu au point 4.1 est utilisé, soit du virus HIV cultivé lorsque le virus cultivé comme au point 4.2 est utilisé. La quantité de p24 est déterminée en utilisant le « HIV-1 p24 Coulter Assay (Beckman Coulter,FL) » et indiquait la présence de 200 ng/ml de p24.

La solution ainsi obtenue est ensuite centrifugée à basse vitesse et le culot est écarté afin d'éliminer le zinc éjecté et précipité. Le surnageant contenant le virus inactivé est conservé pour les étapes ultérieures.

### 4.3 Dégradation de l'ADA par la cystéine.

La composition virale ainsi obtenue contenant une concentration en p24 de 200 ng/ml est ensuite traitée par une concentration de 0,5mM de cystéine (N-Acetyl-L-cystéine Grade, ≥99% (TLC), Sigma Aldrich USA). De cette façon, l'éventuel reliquat d'ADA est réduit en biurée par la cystéine qui est un donneur de protons.

### 4.4 Mesure de l'inactivité des virus traités à l'ADA micronisé

L'inactivité des virus est controlée par la méthode du TCID 50 mentionné précédemment.

Pour la solution contenant les virus inactivés et traitée à la cystéine, on a obtenu réduction de l'infectivité de 5 logarithmes illustrant une perte totale du potentiel infectieux de la composition de virus inactivé selon l'invention.

### Exemple 5.- Production du virus de l'hépatite C inactivé par de l'ADA micronisé

### 5.1 Production de virus de l'hépatite C

On utilise des cellules hépatiques saines provenant de banques d'organes qui ont fourni des tissus hépatiques sains pour d'éventuelles transplantations, mais qui, pour des raisons technologiques mais non médicales, n'ont pu servir.

Les lobes hépatiques encapsulés ainsi obtenus sont traités par une méthode de Segnen modifiée permettant d'envisager un rendement suffisant pour disposer de réserves adéquates d'hépatocytes.

Les fragments hépatiques (lobes de 150g à 500g) sont appareillés avec des cathéters permettant la perfusion de trois à cinq vaisseaux hépatiques.

Le fragment est alors perfusé durant 20 à 30 minutes à 37°C par une solution saline exempte de calcium dite de Hanks (Hank's Balanced Salt Solution (HBSS) 137 mM-NaCl, 5,4 mM KCl, 0,5 mM MgCl₂, 0,4 mM MgSO₄, 0,33 mM Na₂HPO₄, 0,44 mM K₂HPO₄, 5,5 mM D-glucose) additionnée de 0,5mM d'acide éthylène glycol tétraacétique (EGTA).

Toujours à 37°C, la perfusion est remplacée par du milieu selon Leibovitz L-15 (33) contenant 0,05% de collagénase pendant 30 minutes provoquant ainsi la digestion des liens intercellulaires.

Au terme des 30 min, les cathéters sont sectionnés et la biopsie est transférée, toujours stérilement, dans un récipient où sa capsule est incisée. Elle est agitée avec précaution dans le milieu L15- réfrigéré (4°C) sur glace de façon à favoriser la dispersion des hépatocytes.

La suspension cellulaire obtenue est ensuite filtrée sur des tamis stériles en acier inoxydable pour se débarrasser de fragments hépatiques trop gros, afin d'obtenir une taille de fragments de 100 µM. La suspension cellulaire obtenue est ensuite filtrée sur une compresse en nylon toujours stérilement pour obtenir des cellules d'une taille de 60 µM. Le résidu filtré en suspension est réparti en fractions aliquotes dans des tubes de 250 ml et est centrifugé trois fois à 40 G durant 3 minutes à 4°C.

Après la dernière centrifugation, les cellules provenant des fractions aliquotes sont resuspendues, dans un milieu HypoThermosol-FRS® [Solutions BioLife, Inc] dans un seul tube et placé sur de la glace.

Les cellules en suspension ainsi obtenues sont à nouveau centrifugées à 700 tours/minute (rpm) durant 5 minutes à 4°C, le surnageant est enlevé et les cellules sont lavées trois fois avec la solution de lavage dite de Hanks « Hanks Wash » [(53,6 mM KCI 0,4 g/l; 4,4 mM KH₂PO₄ 0,06 g/l; 1,37 M NaCl 8 g/l; 3,4 mM Na₂HPO₄ 0,048 g/l; 20 mL CaCl₂ (2 molaire)].

Les cellules centrifugées et lavées sont remises en suspension dans un milieu minimum essentiel pour la culture, dans des puits sur plaques, des cellules hépatiques (DMEM) (500 mL DMEM high glucose (4,5g/l) supplémenté par 20 % de sérum foetal de bovin).

Dans des plaques à 24 puits, les puits sont remplis à raison de 0,625 x 10⁶ hépatocytes/ml diluées dans environ 10 ml de collagène de type 1 (Collagen I, rat tail-BD Cat. #354236) - (sous forme d'un liquide contenant 0,02 N d'acide acétique, concentration 50 mg/ml) en manière telle que les puits et leurs couvercles en soient couverts.

Les plaques ainsi préparées sont maintenues 1 heure à température ambiante (22°C).

A ce moment, la solution de collagène est retirée et les puits sont rincés avec une solution saline tamponnée au phosphate (PBS) (10 mM Na₂HPO₄, 2 mM KH₂PO₄, 137 mM NaCl, 2,7 mM KCl, pH 7,4) et supplémenté avec le milieu de culture HPM additionné de 20% de sérum de veau foetal. (Adapté aux hépatocytes de toutes les espèces).

Lorsque les hépatocytes sont confluents (environ 18 heures) le HPM est remplacé par un milieu spécifique aux hépatocytes 500 ml DMEM high glucose (4,5g/l) ; 30 mg L-méthionine; 104 mg L-leucine; 33,72 mg L-ornithine; 200 mL of 5mM dexaméthasone; 3 mg d'insuline).

Des fractions aliquotes sont prélevées au milieu de puits de manière aléatoire au bout de 17 h et le nombre d'hépatocytes non adhérent est estimé en mesurant l'activité lactate déshydrogénase après lyse des cellules avec du tampon phosphate salin tel que décrit ci-dessus PBS contenant 0,2 % de Triton X-100. Les puits dans lesquels il y a moins de 85% de cellules adhérentes sont écartés. De même, les puits dans lesquels il y a plus de 5% de cellules en apoptose sont écartés, l'apoptose étant mesurée par la méthode dosant l'annexine V "annexin-V assay" qui détecte le processus d'involution cellulaire extrêmement tôt par le fait que la phospatidylsérine apparaît à la surface cellulaire. Les cultures présentant un taux d'alanine aminotransférase (ALT) supérieur à 3 fois la norme sont également écartées.

Le milieu de culture est changé toutes les 72 heures.

Les hépatocytes ainsi obtenus sont infectés par des virus sauvages de l'hépatite C. Dans ce type de culture d'hépatocytes on peut suivre la cascade réplicative de virus HCV sélectionnés du temps 0 jusqu'à trois semaines après l'infection.

72 heures après l'infection la charge virale monte pour s'établir en plateau entre 100.000 et 1.000.000 de particules durant 3 semaines. Les particules virales sont récupérées à partir du milieu de culture par ultracentrifugation et congelées à -80°C à une concentration par exemple de 2.10¹⁰ particules virales par ml de milieu de congélation (DMEM High).

### 5.2 Production de virus inactivé

La solution stock de particules virales congelées à -80°C est traitée pendant 2 heures à 37°C par une concentration de 0,5 mM d'azodicarbonamide (ADA) micronisé (HPH116 disponible auprès de la société H-Phar Belgique) qui, comme décrit ci-avant, détruit la protéine en doigt de zinc NS5A qui est indispensable au cycle réplicatif de l'HCV.

La solution ainsi obtenue est ensuite centrifugée à basse vitesse et le culot est écarté afin d'éliminer le zinc éjecté et précipité. Le surnageant contenant le virus inactivé est conservé pour les étapes ultérieures.

### 5.3 Dégradation de l'ADA par la cystéine.

La composition virale ainsi obtenue est ensuite traitée par une concentration de 0,5mM de cystéine (N-acétyl-L-cystéine Grade ≥99% (TLC), Sigma Aldrich USA). De cette façon, l'éventuel reliquat d'ADA est réduit en biurée par la cystéine.

### 5.4 Mesure de l'inactivité des virus de l'hépatite C traités à l'ADA micronisé

Une lignée cellulaire Huh 7 (hépatome) (ET) contenant le matériel génétique Hépatite C RNA associé à un témoin Luciférase stable (Luc) a été utilisée pour mesurer l'effet d'inactivation de l'azodicarbonamide micronisé (HPH-116 disponible auprès de H-Phar Belgique).

Le contrôle positif était un traitement par interféron alpha (PBL Biolabs, New Brunswick, NJ USA). Les cellules ET ont été cultivées dans un milieu essentiel Dubecco modifié (DMEM) supplémenté de 10% de sérum de veau foetal, 1% de pénicilline/streptomycine, 1% de glutamine, 5 mg/ml de G418 dans un incubateur a 37°C sous atmosphère contentant 5% de CO2 (tous les réactifs provenaient de Gibco Life Technologies USA). Les cellules ont été tryptinisées (1% trypsine dans de l'EDTA) et réparties dans des plaques contenant 96 puits d'investigations (Costar®). L'activité du gène rapporteur Luciférase (exprimée en unité internationale UI) est proportionnellement liée à la production du RNA de l'hépatite C. La concentration inhibitrice pour 50% de la production de RNA est dans ce modèle de 45µg /ml d'ADA après 72 heures après traitement.

### EXEMPLE 6- Production du virus de l'EBOLA inactivé par de l'ADA micronisé

### 6.1 Production de virus EBOLA:

Des cellules VERO infectées par le virus de l'EBOLA ont été mises en culture dans du milieu essentiel Dubecco modifié (DMEM) supplémenté de 10% de sérum de veau foetal, de 1% de pénicilline/streptomycine, 1% de glutamine, 5 mg/ml de G418 dans un incubateur a 37°C sous atmosphère contentant 5% de CO2 (Tous les réactifs provenaient de Gibco Life Technologies USA) pendant 72 heures.

Le virus a été récolté à partir du surnageant de culture par ultracentrifugation et congelé à -80°C à une concentration par exemple de 2.10¹⁰ particules virales par ml de milieu de congélation.

### 6.2 Production de virus inactivé

La solution stock de particules virales congelées à -80°C a été traitée pendant 2 heures à 37°C par une concentration de 0,5 mM d'azodicarbonamide (ADA) micronisé (HPH116, H-Phar Gosselies Belgium) qui détruit la protéine à doigt de zinc nommée VP30. VP30 a été démontrée indispensable à la réplication du virus EBOLA et intolérante à quelque mutation que ce soit.

La solution ainsi obtenue a ensuite été centrifugée à basse vitesse et le culot a été écarté afin d'éliminer le zinc éjecté et précipité. Le surnageant contenant le virus inactivé a été conservé pour les étapes ultérieures.

### 6.3 Dégradation de l'ADA par la cystéine.

La composition virale ainsi obtenue a ensuite été traitée par une concentration de 0,5mM de cystéine (N-Acetyl-L-cystéine Grade, ≥99% (TLC), Sigma Aldrich USA). De cette façon, l'éventuel reliquat d'ADA est réduit en biurée par la cystéine.

### 6.4 Mesure de l'inactivité des virus EBOLA traités à l'ADA micronisé

Le virus inactivé suivant l'invention a été évalué au niveau de son activité par l'infection de cellules VERO natives mises en culture selon un protocole standard et par l'infection de cellules VERO exprimant la protéine VP30 (transfection des cellules VERO par le gène codant pour la protéine VP30) comme contrôle positif.

Comme attendu les cellules VERO normales infectées n'ont pas produit de nouvelles particules virales tandis que les cellules VERO transfectées par le gène de la VP30 ont produit des virus EBOLA complets.

En outre, selon l'invention, il est également envisagé de doser le zinc libéré pour mesurer l'inactivation des virus étant donné que chaque inactivation des protéines à doigt de zinc relargue un atome de zinc dans le milieu de traitement du virus par l'ADA micronisé.

### EXEMPLE 7.- Production d'arenavirus inactivé par de l'ADA micronisé

### 7.1 Production d'arenavirus:

Des cellules VERO infectées par trois arenavirus différents, le virus LCMV-WE reçu de Peter B. Jahrling (U.S. Army Medical Research Institute for Infectious Diseases, Fort Detrick, Frederick, Md.) , JUNV et TCRV ont été mises en culture séparément dans du milieu essentiel Dubecco modifié (DMEM) supplémenté de 10% de sérum de veau foetal, de 1 % de pénicilline/streptomycine, 1% de glutamine, 5 mg/ml de G418 dans un incubateur a 37°C sous atmosphère contentant 5% de CO2 (Tous les réactifs provenaient de Gibco Life Technologies USA) pendant 72 heures, jusqu'à obtenir 10⁷ virus/ml de milieu de culture.

Le virus a été récolté à partir du surnageant de culture par ultracentrifugation et congelé à -80°C à une concentration par exemple de 2.10¹⁰ particules virales par ml de milieu de congélation.

### 7.2 Production de virus inactivé

La solution stock de particules virales congelées à -80°C a été traitée pendant 2 heures à 37°C par une concentration de 0,5 mM d'azodicarbonamide (ADA) micronisé (HPH116, H-Phar Gosselies Belgium) qui, détruit la protéine à motif en doigt de zinc nommée PZ. La réplication des arenavirus est conditionnée par une protéine à doigt de zinc appelée protéine Z. Les protéines ribosomales de la cellule-hôte interagissent avec la protéine Z et les ribosomes, étroitement associés au génome viral, intervenant dans la traduction des ARNm viraux.

La solution ainsi obtenue a ensuite été centrifugée à basse vitesse et le culot a été écarté afin d'éliminer le zinc éjecté et précipité. Le surnageant contenant le virus inactivé a été conservé pour les étapes ultérieures.

### 7.3 Dégradation de l'ADA par la cystéine.

La composition virale ainsi obtenue a ensuite été traitée par une concentration de 0,5mM de cystéine (N-acétyl-L-cystéine Grade ≥ 99% (TLC), Sigma Aldrich USA). De cette façon, l'éventuel reliquat d'ADA est réduit en biurée par la cystéine.

### 7.4 Mesure de l'inactivité des Arenavirus traités à l'ADA micronisé

Le virus inactivé suivant l'invention a été évalué au niveau de son activité par une mesure d'absorption d'UV. La protéine PZ du virus actif présente une structure cyclique dans laquelle se trouve l'atome de zinc. Cette protéine absorbe l'UV dans sa structure cyclique à une longueur d'onde de 950 nm. L'éjection de l'atome de zinc conduit à la déstabilisation de cette structure cyclique de la protéine Z virale et a pour résultat que la protéine PZ n'absorbe plus l'UV à la même longueur d'onde. La mesure d'absorption d'UV à la longueur d'onde de 200 nm permet donc de mesurer l'inactivité de la protéine PZ.

Dans le cas présent, les virus JUNV et TCRV traités à l'ADA permettaient d'obtenir respectivement des valeurs d'IC50 de 7,6 et 5,3 µM tandis que le virus LCMV-WE traité à l'ADA permettait d'atteindre des valeurs d'IC50 de 27,9 µM respectivement.

### EXEMPLE 8.- Vaccin par administration directe

### 8.1 Préparation de vaccin par administration directe par injection

### Vaccin à base de virus autologue

On a prélevé 0.5 ml de virus tels qu'obtenus à l'exemple 4.1 (2.10¹⁰ particules virales/ml) et on a exposé ces virus à une concentration de 3 µM d'ADA micronisé (HPH116) pendant 2 heures à 37°C.

On a ensuite ajouté 0,5 ml d'adjuvant incomplet de Freund pour obtenir un volume final de 1 ml. Cette dose vaccinale a été préparée selon les protocoles bien établis dans le domaine et bien entendu, si c'est nécessaire, des excipients de conservation peuvent également être ajoutés. La dose de 1 ml sera ensuite injectée en 4 doses de 0,25 ml (quatre injections réparties sur le corps, épaules et hanches).

### Vaccin à base d'une préparation virale régionale

Des souches du virus VIH-1 sauvages des sous-types A, B et D ont été cultivés sur des lymphocytes de volontaires sains afin d'obtenir dans le milieu de culture RPMI comme déjà décrit plus en détails ci-dessus. Ensuite, les lymphocytes infectés ont ensuite été activés par de la phytohémagglutinine.

Tous les 7 jours, un échantillon de surnageant a été prélevé pour mesurer le taux de p24 et ainsi évaluer la croissance virale. Lorsque le taux de p24 était de 50 µg/ml, la culture de cellules a été centrifugée et le surnageant stocké en lots de stock de virus à -80°C de manière à obtenir, après ultracentrifugation, 2.10¹⁰ particules virales par ml.

On a prélevé 0.5 ml de virus ainsi obtenus (2.10¹⁰ particules virales/ml) et on a exposé ces virus à une concentration de 3 µM d'ADA micronisé (HPH116) pendant 2 heures à 37°C.

On a ensuite ajouté 0,5 ml d'adjuvant incomplet de Freund pour obtenir un volume final de 1 ml. Cette dose vaccinale a été préparée selon les protocoles bien établis dans le domaine et bien entendu, si c'est nécessaire, des excipients de conservation peuvent également être ajoutés. La dose de 1 ml sera ensuite injectée en 4 doses de 0,25 ml (quatre injections réparties sur le corps, épaules et hanches).

Cette préparation vaccinale a bien entendu un but prophylactique ou thérapeutique. De préférence, l'injection est répétée au bout de 4 semaines.

### 8.2 Préparation de vaccin par administration intramuqueuse ou intralymphatique directe

Une dose de TCID 50 de virus HPV inactivé d'une manière analogue à ce qui a été décrit précédemment est réalisée et mise en suspension dans un ovule à introduction vaginale. La composition des ovules est mentionnée au tableau 6.

**Tableau 6.**

| Ingrédients | quantité |
|---|---|
| Virus inactivé selon l'exemple 4.2 | 10⁶ ad 1 ml |
| Sel sodique d'acide hyaluronique | 5 mg |
| Extrait lipidique de Centella asiatica | 60 mg |
| Extrait lipidique de Calendula officinalis | 60 mg |
| Extrait lipidique d'Aloe vera | 60 mg |
| Huile essentielle de Melaleuca | 2 mg |
| Excipients | |

Ces excipients sont des glycérides semi-synthétiques, du BHT (butylhydroxytoluène), du parabène isopropylique, du parabène isobutylique, du parabène butylique et analogues. Ces excipients présentent des effets galéniques particuliers: après liquéfaction de l'ovule à température corporelle, les virus inactivés se distribuent uniformément dans le vagin en l'espace de quelques minutes. Il s'y dépose en formant un film de gel qui se résorbe lentement sur 2-3 jours.Le sel sodique d'acide hyaluronique présente quant à lui un rôle en tant que substrat dans la migration cellulaire.

Ils sont alors pris en charge par les cellules dendritiques de la patiente et le taux d'anticorps sanguin apparaissant envers le HPV atteste de la vaccination.

### EXEMPLE 9.-Préparation d'un vaccin à l'aide de cellules dendritiques isolées.

### 9.1. Culture de cellules dendritiques

Lors d'une prise de sang réalisée dans des tubes citratés chez le sujet porteur du VIH, des monocytes circulant ont été isolés par la méthode de centrifugation par gradient de densité suivant la méthode Ficoll-Hypaque (Eurobio, les Ulis France) (cellules PBMC).

Ces monocytes ont ensuite été lavés 4 fois de manière successive par une solution tampon de Hanks.

Une immunosélection négative a ensuite été réalisée en utilisant un kit commercial (Dynal, great Neck, New York ; USA) en vue d'isoler les monocytes portant le récepteur CD14⁺. 90 % des monocytes isolés sont 90% des cellules portant le récepteur CD14⁺ (Cellules CD14⁺ pures à 90%).

Elles sont alors réparties dans des flacons (Nunc, Roskilde, Danemark) à raison de 10⁶/ml cellules dans un milieu exempt de sérum « serum-free AIM-V medium » (Life Technologies, Grand Island, New York ; USA.)

Ces cellules sont ainsi cultivées durant 5 jours dans le milieu AIM-V supplémenté de 250 ng de facteur de stimulation des colonies de granulocytes macrophages (GM-CSF) (R&D Systems, Minneapolis, USA) et de 100 ng d'interleukine 4 (IL-4) (R&D Systems, Minneapolis, USA) par millilitre.

Après ce traitement et les 5 jours de culture, les cellules non adhérentes sont identifiées comme étant à plus de 90% des cellules dendritiques immatures (DC) en se basant sur leur morphologie (observation microscopique) et sur les récepteurs membranaires exprimés (CD1a, CD11c, CD40, CD80, CD86, CD4 et HLA-DR mis en évidence par immunofluorescence directe en cytométrie de flux).

### 9.2 Induction d'une réponse spécifique au virus du patient par les cellules dendritiques du patient

Les cellules CD immatures ainsi obtenues (1.10⁶ cellules/ml) ont été sont impulsées par le HIV autologue inactivé par l'ADA obtenu à l'exemple 4 (point 4.2) (50ng de p24) suivant un rapport 50ng de p24/10⁶ cellules) durant 2 heures à température ambiante.

Les cellules CD ainsi impulsées par l'antigène HIV autologue inactivé (CD-HIV-impulsées) sont alors lavées trois fois consécutivement par la solution tampon de Hanks.

Les CD-HIV-impulsées sont mises en culture (37°C- 5% CO₂) dans le milieu AIM-V supplémenté de 100ng de GM-CSF, de 5 ng d'IL-4, 50 ng TNF-α (R&D Systems USA) et de 1.000 Unités d'inteferon alpha 2b (IFN-α-2b) (Schering-Plough, Brinny, Ireland) par millilitre durant trois jours.

Les cellules dendritiques ainsi cultivées en présence de virus inactivé selon l'invention ont internalisé le virus inactivé et sont alors dites chargées ou stimulées et prêtes à être ré-injectées par voie d'administration sous-cutanée, intradermique ou intralymphatique. Ces cellules dendritiques sont activées par l'action de cytokines et expriment à leur surface des antigènes (peptides) spécifiques du virus inactivé internalisé ayant permis de les stimuler.

Il est bien entendu que la présente invention n'est en aucune façon limitée aux formes de réalisations décrites ci-dessus et que bien des modifications peuvent y être apportées sans sortir du cadre des revendications annexées.

Par exemple, au lieu d'utiliser du virus autologue, cultivé ou non, pour fabriquer un vaccin à virus inactivé, il est également possible d'utiliser une culture de virus HIV à grande échelle. Dans ce cas, le virus est obtenu par une production par des cellules. Par exemple, le virus HIV peut être cultivé à grande échelle comme décrit dans EP 0 524 961. Le virus cultivé sera également récupéré du surnageant de culture par ultracentrifugation et congelé à -80°C à une concentration par exemple de 2.10¹⁰ particules virales par ml de milieu de congélation.

## Revendications

1. Composition pour son utilisation en tant que vaccin à base d'au moins une souche de virus exhibant au moins une protéine à motif(s) en doigt de zinc PZ, comprenant ces virus dans un état inactivé, ces virus étant entiers et présentant une enveloppe virale intacte après inactivation,
**caractérisée en ce que** ledit zinc de ladite protéine PZ a été éjecté de ladite au moins une protéine PZ par une réaction d'oxydoréduction avec de l'azodicarbonamide et **en ce que** ladite composition comprend en outre de la biurée formée à partir de l'azodicarbonamide au cours de ladite réaction d'oxydoréduction, ainsi qu'éventuellement un reliquat d'azodicarbonamide n'ayant pas réagi.

2. Composition pour son utilisation suivant la revendication 1, **caractérisée en ce qu'**elle contient en outre un donneur de protons pharmaceutiquement compatible, en particulier un réducteur physiologique, dans un état déprotonisé qui est également pharmaceutiquement compatible et qui résulte d'une réaction d'oxydoréduction additionnelle avec ledit reliquat d'azodicarbonamide n'ayant pas réagi, ainsi qu'éventuellement
- un reste de donneur de protons n'ayant pas réagi.

3. Composition pour son utilisation suivant la revendication 2, **caractérisée en ce que** le donneur de protons est choisi parmi le groupe constitué de la cystéine, du glutathion, du nicotinamide phosphate (NADPH) et de leurs dérivés et mélanges et **en ce que** le donneur de protons déprotonisé est respectivement la cystine, le glutathion oxydé, le NADP⁺ ou un dérivé de ceux-ci.

4. Composition pour son utilisation suivant l'une ou l'autre des revendications 1 à 3, **caractérisée en ce que** ladite au moins une souche de virus est choisie parmi le groupe constitué des rétrovirus, des arénavirus, des filovirus, des hépavirus, des papillomavirus et des virus d'herpès qui exhibent des protéines PZ.

5. Composition suivant l'une quelconque des revendications 1 à 4, **caractérisée en ce qu'**elle comprend en outre des cellules immunocompétentes isolées qui ont éventuellement au moins partiellement internalisé lesdits virus inactivés.

6. Procédé de préparation d'une composition vaccinale suivant l'une quelconque des revendications 1 à 5, comprenant
- une mise en contact d'au moins une souche de virus exhibant au moins une protéine à motif(s) en doigt de zinc PZ avec de l'azodicarbonamide micronisé,
- une pénétration de l'azodicarbonamide micronisé à travers l'enveloppe virale desdits virus en laissant celle-ci intacte,
- une inactivation des virus par une réaction d'oxydoréduction entre l'azodicarbonamide micronisé et ladite au moins une protéine PZ, résultant en une éjection du zinc à partir de ladite protéine PZ et en une décomposition au moins partielle de l'azodicarbonamide en biurée,
- une mise en contact des virus inactivés avec un donneur de protons pharmaceutiquement acceptable, en particulier un réducteur physiologique, qui est aussi pharmaceutiquement acceptable à l'état déprotonisé, et
- une réaction d'oxydoréduction additionnelle entre ce donneur de protons et un éventuel reliquat d'azodicarbonamide n'ayant pas réagi, ce qui déprotonise ledit donneur de protons et décompose ledit reliquat en biurée. 6.

7. Procédé suivant la revendication 6, **caractérisé en ce que** le donneur de protons est choisi dans le groupe constitué de la cystéine, du glutathion, du nicotinamide phosphate et de leurs dérivés et mélanges et **en ce que** le donneur de protons déprotonisé est respectivement la cystine, le glutathion oxydé ou le NADP⁺ ou un dérivé de ceux-ci.

8. Procédé suivant l'une quelconque des revendications 6 à 7, **caractérisé en ce que** l'azodicarbonamide micronisé se présente sous la forme de particules qui sont dépourvues d'additif et qui montrent une taille inférieure à 10 µm, de préférence à 8 µm, en particulier à 5 µm.

9. Procédé suivant l'une quelconque des revendications 6 à 8, **caractérisé en ce qu'**il comprend en outre une élimination du zinc éjecté.

10. Procédé suivant la revendication 9, **caractérisé en ce qu'**il comprend en outre un isolement des virus inactivés.

11. Procédé suivant l'une quelconque des revendications 6 à 10. **caractérisé en ce qu'**il comprend en outre une impulsion de cellules immunocompétentes isolées par ladite composition contenant des virus inactivés par azodicarbonamide, une mise en culture ex vivo des cellules impulsées et une expression à leur surface d'antigènes spécifiques desdits virus inactivés.

12. Utilisation d'une composition suivant l'une quelconque des revendications 1 à 5, pour la fabrication d'un vaccin prophylactique ou thérapeutique contre au moins une infection par au moins un desdits virus exhibant au moins une protéine à motif(s) en doigt de zinc PZ.

13. Utilisation suivant la revendication 12, dans laquelle le vaccin produit est un vaccin administrable par injection ou par voie transmuqueuse, orale, génitale ou rectale.

## Patentansprüche

1. Zusammensetzung, die dazu bestimmt ist, als Impfstoff verwendet werden, auf Grundlage mindestens eines Virenstamms, der mindestens ein Protein PZ mit Zinkfingerdomäne präsentiert, wobei sie diese Viren in einem inaktivierten Zustand enthält, wobei diese Viren vollständig sind und nach der Inaktivierung eine intakte Virushülle aufweisen,
**dadurch gekennzeichnet, dass** das Zink des Proteins PZ durch eine Redoxreaktion mit Azodicarbonamid aus dem mindestens einen Protein PZ verdrängt wurde, und dadurch, dass die Zusammensetzung darüber hinaus Diharnstoff umfasst, welcher im Laufe der Redoxreaktion aus dem Azodicarbonamid gebildet wurde, sowie möglicherweise eine verbleibende Menge an nicht umgesetztem Azodicarbonamid.

2. Zusammensetzung, die dazu bestimmt ist, gemäß Anspruch 1 verwendet zu werden, **dadurch gekennzeichnet, dass** sie darüber hinaus einen pharmazeutisch verträglichen Protonenspender enthält, insbesondere ein physiologisches Reduktionsmittel, das in einem deprotonierten Zustand vorliegt, welcher ebenfalls pharmazeutisch verträglich ist, und welcher bei einer zusätzlichen Redoxreaktion mit der verbleibenden Menge an nicht umgesetztem Azodicarbonamid erhalten wurde, sowie möglicherweise
- einen Rest an nicht umgesetztem Protonenspender.

3. Zusammensetzung, die dazu bestimmt ist, gemäß Anspruch 2 verwendet zu werden, **dadurch gekennzeichnet, dass** der Protonenspender aus der Gruppe ausgewählt ist, die aus Cystein, Glutathion, Nicotinamidphosphat (NADPH) und deren Derivaten und Mischungen besteht, und dadurch, dass es sich bei dem deprotonierten Protonenspender um Cystin, oxidiertes Glutathion, NADP⁺ beziehungsweise ein Derivat derselben handelt.

4. Zusammensetzung, die dazu bestimmt ist, gemäß einem der Ansprüche 1 bis 3 verwendet zu werden, **dadurch gekennzeichnet, dass** der mindestens eine Virenstamm aus der Gruppe ausgewählt ist, die aus den Retroviren, den Arenaviren, den Filoviren, den Hepaviren, des Papillomaviren und den Herpesviren besteht, wobei diese PZ-Proteine präsentieren.

5. Zusammensetzung dazu bestimmt ist, gemäß einem beliebigen der Ansprüche 1 bis 4 verwendet zu werden, **dadurch gekennzeichnet, dass** sie darüber hinaus isolierte immunkompetente Zellen umfasst, die möglicherweise die inaktivierten Viren mindestens teilweise in sich aufgenommen haben.

6. Verfahren zur Herstellung einer Impfstoffzusammensetzung nach einem beliebigen der Ansprüche 1 bis 5, welches Folgendes umfasst
- Inkontaktbringen mindestens eines Virenstamms, der mindestens ein Protein PZ mit Zinkfingerdomäne präsentiert, mit mikronisiertem Azodicarbonamid,
- Eindringen des mikronisierten Azodicarbonamids durch die Virenhülle der Viren, wobei diese intakt bleibt,
- Deaktivierung der Viren durch eine Redoxreaktion zwischen dem mikronisiertem Azodicarbonamid und dem mindestens einen Protein PZ, was zu einer Verdrängung des Zinks aus dem Protein PZ und zu einem mindestens teilweisen Zerfall des Azodicarbonamid zu Diharnstoff führt,
- Inkontaktbringen der inaktivierten Viren mit einem pharmazeutisch unbedenklichen Protonenspender, insbesondere einem physiologischen Reduktionsmittel, welches auch im deprotonierten Zustand pharmazeutisch unbedenklich ist, und
- zusätzliche Redoxreaktion zwischen diesen Protonenspender und einer möglicherweise verbleibenden Menge an nicht umgesetztem Azodicarbonamid, wodurch der Protonenspender deprotoniert wird und die verbleibende Menge zu Diharnstoff zerfällt.

7. Verfahren nach Anspruch 6, **dadurch gekennzeichnet, dass** der Protonenspender aus der Gruppe ausgewählt ist, die aus Cystein, Glutathion, Nicotinamidphosphat (NADPH) und deren Derivaten und Mischungen besteht, und dadurch, dass es sich bei dem deprotonierten Protonenspender um Cystin, oxidiertes Glutathion beziehungsweise NADP⁺ oder ein Derivat derselben handelt.

8. Verfahren nach einem beliebigen der Ansprüche 6 bis 7, **dadurch gekennzeichnet, dass** das mikronisierte Azodicarbonamid in Form von Partikeln vorliegt, die keinerlei Additiv aufweisen und die eine Größe von weniger als 10 µm, vorzugsweise als 8 µm, insbesondere als 5 µm zeigen.

9. Verfahren nach einem beliebigen der Ansprüche 6 bis 8, **dadurch gekennzeichnet, dass** es darüber hinaus eine Entfernung des verdrängten Zinks umfasst.

10. Verfahren nach Anspruch 9, **dadurch gekennzeichnet, dass** es darüber hinaus eine Isolierung der inaktivierten Viren umfasst.

11. Verfahren nach einem beliebigen der Ansprüche 6 bis 10, **dadurch gekennzeichnet, dass** es darüber hinaus eine Pulsbehandlung der isolierten immunkompetenten Zellen mit der Zusammensetzung, welche mittels Azodicarbonamid inaktivierte Viren enthält, umfasst sowie eine Anzüchtung ex vivo der pulsbehandelten Zellen und eine Expression von Antigenen, die für die inaktivierten Viren spezifisch sind, an deren Oberfläche.

12. Verwendung einer Zusammensetzung nach einem beliebigen der Ansprüche 1 bis 5, um einen vorbeugenden oder therapeutischen Impfstoff gegen mindestens eine Infektion mit mindestens einem der Viren herzustellen, welche mindestens ein Protein PZ mit Zinkfingerdomäne präsentieren.

13. Verwendung nach Anspruch 12, wobei es sich bei dem hergestellten Impfstoff um einen Impfstoff handelt, der durch Injektion oder über die Schleimhäute oder auf oralem, genitalem oder rektalem Wege verabreicht werden kann.

## Claims

1. A composition for its use as a vaccine, based on at least one virus strain exhibiting a protein PZ with zinc finger pattern(s), comprising these viruses in an inactivated state, these viruses being entire and having an intact virus envelope after inactivation,
**characterized in that** said zinc of said protein PZ has been ejected from said at least one protein PZ by an oxidation-reduction reaction with azodicarbonamide and **in that** said composition further comprises biurea formed from azodicarbonamide during the oxidation-reduction reaction, as well as possibly a remainder of unreacted azodicarbonamide.

2. The composition for its use according to claim 1, **characterized in that** it further contains a pharmaceutically compatible proton donor, in particular a physiological reducing agent, in a deprotonated state which is also pharmaceutically compatible and which results from an additional oxidation-reduction reaction with said remainder of unreacted azodicarbonamide, as well as possibly
- an unreacted proton donor remainder.

3. The composition for its use according to claim 2, **characterized in that** the proton donor is selected from the group consisting of cysteine, glutathion, nicotinamide phosphate (NADPH) and derivatives and mixtures thereof and **in that** the deprotonated proton donor is cystine, oxidized glutathione, NADP⁺ or a derivative thereof, respectively.

4. The composition for its use according to either one of claims 1 to 3, **characterized in that** said at least one virus strain is selected from the group consisting of retroviruses, arenaviruses, filoviruses, hepaviruses, papillomaviruses, and herpes viruses which exhibit PZ proteins.

5. The composition for its use according to any of claims 1 to 4, characterizing that it further comprises isolated immunocompetent cells which have possibly at least partly internalized said inactivated viruses.

6. A method for preparing a vaccine composition according to any of claims 1 to 5, comprising
- putting at least one virus strain exhibiting at least one protein PZ with zinc finger pattern(s) in contact with micronized azodicarbonamide,
- penetration of the micronized azodicarbonamide through the viral envelope of said viruses by leaving the latter intact,
- inactivation of the viruses by an oxidation-reduction reaction between the micronized azodicarbonamide and said at least one protein PZ resulting in an ejection of zinc from said protein PZ and in an at least partial decomposition of the azodicarbonamide into biurea,
- putting the inactivated viruses in contact with a pharmaceutically acceptable proton donor, in particular a physiological reducing agent, which is also pharmaceutically acceptable to the deprotonated state, and
- an additional oxidation-reduction reaction between this proton donor and a possible unreacted azodicarbonamide remainder, which deprotonates said proton donor and decomposes said remainder into biurea.

7. The method according to claim 6, characterizing that the proton donor is selected from the group consisting of cysteine, glutathion, nicotinamide phosphate and derivatives and mixtures thereof and in that the deprotonated proton donor is cystine, oxidized glutathione, NADP⁺ or a derivative thereof, respectively.

8. The method according to any of claims 6 to 7, **characterized in that** the micronized azodicarbonamide appears as particles which are without any additive and which exhibit a size of less than 10 µm, preferably less than 8 µm, in particular 5 µm.

9. The method according to any of claims 6 to 8, characterizing that it further comprises removal of the ejected zinc.

10. The method according to claim 9, **characterized in that** it further comprises isolation of the inactivated viruses.

11. The method according to any of claims 6 to 10, characterizing that it further comprises a pulse of immunocompetent cells isolated by said composition containing viruses inactivated by azodicarbonamide, cultivation ex vivo of pulsed cells and expression at their surface of specific antigens of said inactivated viruses.

12. The use of a composition according to any of claims 1 to 5, for manufacturing a prophylactic or therapeutic vaccine against at least one infection by at least one of said viruses exhibiting at least one protein PZ with zinc finger pattern(s).

13. The use according to claim 12, wherein the produced vaccine is a vaccine which may be administered by injection or via a transmucosal, oral, genital or rectal route.
